# FASCICULE DE BREVET EUROPEEN

(11) **EP 3 967 402 B1**
(45) Date de publication et mention de la délivrance du brevet: **18.03.2026**
(21) Numéro de dépôt: 21193218.1
(22) Date de dépôt: 26.08.2021
(51) Int. Cl.: B01L 3/00, C12Q 1/6844, C12Q 1/6804

(54) **PROCÉDÉ DE DÉTECTION D'ESPÈCES BIOLOGIQUES PRÉSENTES DANS UN ÉCHANTILLON BIOLOGIQUE**
VERFAHREN ZUM NACHWEIS VON BIOLOGISCHEN SPEZIES IN EINER BIOLOGISCHEN PROBE
METHOD FOR DETECTING OF BIOLOGICAL SPECIES CONTAINED IN A BIOLOGICAL SAMPLE

(30) Priorité: 11.09.2020 FR 2009214
(43) Date de publication de la demande: 16.03.2022
(73) Titulaire: Commissariat à l'Energie Atomique et aux Energies Alternatives, 75015 Paris (FR)
(72) Inventeur: CUBIZOLLES, Myriam-Laure, 38054 GRENOBLE Cedex 09 (FR); BOURDAT, Anne-Gaëlle, 38054 GRENOBLE CEDEX 09 (FR); SAVONNET, Maud, 38054 GRENOBLE CEDEX 09 (FR)
(74) Mandataire: INNOV-GROUP

(56) Documents cités:
- EP-A1- 3 222 989
- EP-A1- 3 629 000
- US-A1- 2018 163 270
- YADONG TANG ET AL: "Microfluidic device with integrated microfilter of conical-shaped holes for high efficiency and high purity capture of circulating tumor cells", SCIENTIFIC REPORTS, vol. 4, no. 1, 13 August 2014 (2014-08-13), XP055660349, DOI: 10.1038/srep06052
- KONG JANAY ET AL: "ISOTHERMAL EXPONENTIALLY AMPLIFIED IMMUNOASSAY WITH SIZE-SELECTIVE OLIGONUCLEOTIDE BACKGROUND REDUCTION", 1 October 2015 (2015-10-01), XP055805020, Retrieved from the Internet <URL:https://www.rsc.org/images/LOC/2015/PDFs/Papers/0931_W.207c.pdf>

## Description

### Domaine technique de l'invention

La présente invention se rapporte à un procédé de préparation d'un échantillon biologique et de détection d'espèces biologiques présentes dans cet échantillon biologique.

### Etat de la technique

La demande de brevet EP3222989A1 décrit un dispositif permettant de lyser des espèces biologiques présentes dans un échantillon, notamment pour extraire des molécules d'ADN en vue d'une détection par amplification de type PCR. En revanche, cette demande de brevet est limitée à la détection par amplification de type biomoléculaire après une lyse des espèces biologiques.

Même si la préparation d'un échantillon pour une détection immunologique et la préparation d'un échantillon pour une extraction d'ADN sont souvent très différentes, la demande de brevet EP3629000A1 présente pour sa part une méthode de préparation qui permet, à partir d'un seul dispositif et d'un même échantillon, de réaliser une préparation d'un premier échantillon en vue d'une reconnaissance de type immunologique et une préparation d'un deuxième échantillon en vue d'une réaction par amplification.

Cependant, cette dernière solution ne permet que de préparer les deux échantillons et oblige à sortir le premier échantillon du dispositif en vue de la reconnaissance de type immunologique et à sortir le deuxième échantillon du dispositif en vue de la détection par amplification.

La demande de brevet US2018/163270A1 décrit pour sa part la méthode dite "immuno-PCR". Cette méthode est censée combiner les avantages d'une méthode de type immunologique telle que ELISA (pour "Enzyme-Linked Immunosorbent Assay") et ceux de la méthode PCR (pour "Polymerase Chain Reaction"). Cette méthode apparue en 1992 permet d'augmenter la sensibilité de détection par rapport à la méthode ELISA classique. Classiquement, elle repose sur l'utilisation d'un réactif obtenu par le couplage d'un anticorps, qui est destiné à la reconnaissance de la cible, avec une séquence oligonucléotidique.

Yadong Tang et al. (SCIENTIFIC REPORTS, vol. 4, no. 1, 13-08-2014) décrit un procédé de détection par immunofluorescence d'un échantillon biologique comprenant des espèces biologiques d'intérêt, mis en œuvre dans un dispositif qui comporte un filtre ayant une porosité adaptée pour retenir le matériel biologique à analyser.

Le but de l'invention est de proposer un procédé adapté pour réaliser une préparation de l'échantillon, avantageusement sans étape de lyse des espèces biologiques présentes dans l'échantillon, en vue d'une reconnaissance de type immunologique (capture sélective) et d'une détection par amplification biomoléculaire, ce procédé étant rapide, fiable, sans fractionner l'échantillon et en engageant toutes les cibles présentes dans l'échantillon.

### Exposé de l'invention

Ce but est atteint par un procédé de détection d'espèces biologiques cibles dans un échantillon biologique, ledit procédé étant mis en œuvre dans un système de détection qui comporte un dispositif de préparation et des moyens de détection, ledit dispositif de préparation comportant :
- Un boîtier comportant au moins une ouverture,
- Un premier canal ménagé dans ledit boîtier,
- Un deuxième canal ménagé dans ledit boîtier,
- Une chambre dans laquelle débouche le premier canal et le deuxième canal,
- Un filtre séparant ladite chambre en deux espaces distincts de manière à définir un premier espace dans lequel débouche ledit premier canal d'injection et un deuxième espace dans lequel débouche ledit deuxième canal, ledit filtre ayant une porosité adaptée pour retenir ledit matériel biologique à analyser,

Ledit procédé comportant des étapes de :
- Injection de l'échantillon par le premier canal pour effectuer une concentration des espèces biologiques cibles dans le premier espace de la chambre, mise en œuvre par filtration,
- Injection par le premier canal d'une première solution de mise en condition de réaction par reconnaissance sélective,
- Injection par le premier canal d'une solution d'un premier réactif hybride comportant des molécules de reconnaissance sélective des espèces biologiques cibles, ces molécules de reconnaissance sélective étant liées à une séquence oligonucléotidique adaptée à une initiatior d'une réaction d'amplification biomoléculaire,
- Injection par le premier canal d'une deuxième solution de mise en condition de réaction par amplification biomoléculaire,
- Injection par le premier canal d'une solution d'un réactif adapté à une amplification biomoléculaire,
- Détection des espèces cibles directement dans le dispositif par amplification biomoléculaire. de la séquence oligonucléotidique des molécules de reconnaissance sélective.

Selon une particularité, le procédé comporte une étape de lavage des espèces biologiques cibles concentrées dans l'espace inférieur du dispositif, après l'étape d'injection de l'échantillon.

Selon une autre particularité, le procédé comporte une étape de lavage des espèces biologiques cibles mise en œuvre après la réaction de reconnaissance sélective.

Selon une autre particularité, l'étape de détection par amplification est mise en œuvre par fluorescence, colorimétrie, pH métrie, turbidimétrie, imagerie sans lentille.

Selon une autre particularité, le premier réactif comporte des molécules de capture de type anticorps ou aptamère ou de type MIP.

Selon une autre particularité, le deuxième réactif comporte des amorces de réaction par amplification.

### Brève description des figures

D'autres caractéristiques et avantages vont apparaître dans la description détaillée qui suit, faite en liaison avec les figures listées ci-dessous :
[Fig. 1]

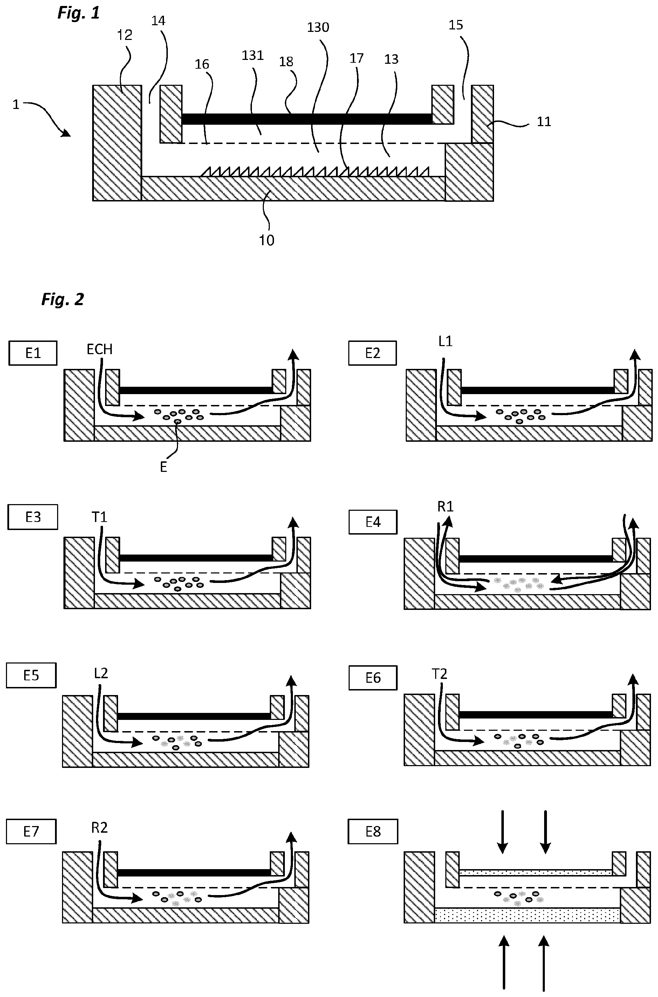

   - La figure 1 représente le dispositif employé pour la préparation de l'échantillon et la détection des espèces biologiques.
[Fig. 2]
   - La figure 2 illustre les différentes étapes E1 à E8 du procédé de l'invention, mises en œuvre dans le dispositif de la figure 1.
[Fig. 3]

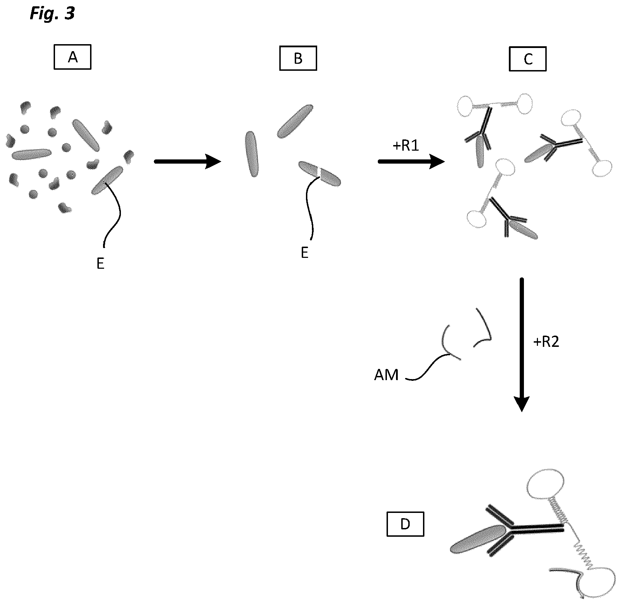

   - La figure 3 illustre également le principe de préparation de l'échantillon et de capture des espèces biologiques cibles préalablement à leur détection.

### Description détaillée d'au moins un mode de réalisation

L'invention concerne un procédé mis en œuvre dans un dispositif qui permet d'enchaîner la préparation d'un échantillon pour une détection par capture sélective et une détection par amplification biomoléculaire.

La solution permet de détecter la présence d'espèces biologiques cibles dans un échantillon biologique.

L'échantillon biologique se présente par exemple sous la forme d'un fluide.

Par espèces biologiques, on entend notamment des micro-organismes, des cellules, des spores, des virus, des toxines...

Par fluide, on entend notamment un liquide, un gaz... Le liquide pourra présenter différents degrés de viscosité et pourra par exemple se présenter sous la forme d'une pâte ou d'un gel.

Le procédé permet notamment de détecter les espèces biologiques cibles présentes dans l'échantillon en utilisant un même dispositif, que ce soit par réaction de type capture sélective ou par réaction d'amplification biomoléculaire.

Selon un aspect particulier de l'invention, le dispositif employé est tel que décrit ci-dessous, en liaison avec la figure 1.

Le dispositif de préparation peut être inclus dans un système de détection plus global, intégrant des moyens de détection adaptés à la mise en œuvre d'une détection par amplification. De manière non limitative, ces moyens de détection sont par exemple choisis parmi, du matériel optique pour détection de fluorescence, colorimétrie, un pH mètre, du matériel de détection par imagerie sans lentille, du matériel de détection par turbidimétrie...

Le dispositif 1 comporte un boîtier comprenant une paroi inférieure 10, une paroi latérale 11 et une paroi supérieure 12. Toutes les parois du boîtier seront préférentiellement réalisées dans un même matériau. Ce matériau sera notamment apte à pouvoir subir un chauffage dans une fourchette de température comprise entre 20°C et 100°C. Préférentiellement, certaines parois du boîtier seront réalisées dans un matériau transparent. Préférentiellement, le matériau employé sera un plastique, par exemple de type PMMA (Poly(Méthacrylate de Méthyle)) ou COC (Cyclic Olefin Copolymer).

Le dispositif 1 comporte une chambre 13 ménagée dans le boîtier. Cette chambre représente l'emplacement dans lequel sont effectuées à la fois la purification/concentration et la détection des espèces biologiques cibles. La chambre 13 est fermée vers le bas par la paroi inférieure du boîtier.

Le dispositif comporte un premier canal 14 ménagé dans le boîtier et agencé pour injecter des fluides dans la chambre ou pour évacuer des fluides en dehors de la chambre. Le premier canal 14 comporte une première extrémité comportant une ouverture ménagée par exemple à travers la paroi supérieure 12 du boîtier et une deuxième extrémité qui débouche dans ladite chambre 13. La première extrémité du premier canal 14 est par exemple agencée verticalement et sa deuxième extrémité débouche par exemple horizontalement dans la chambre 13. La première extrémité du premier canal est par exemple évasée pour y appliquer le cône d'une pipette ou sera adaptée au type de dispositif employé pour injecter le fluide dans le dispositif. A titre d'exemple, il peut s'agir d'une ouverture présentant un embout de type luer pour y connecter une seringue ou adaptée pour y connecter un circuit fluidique tel que celui qui sera décrit ci-après.

Le dispositif comporte un deuxième canal 15 ménagé dans le boîtier. Ce deuxième canal 15 comporte également une première extrémité qui communique avec l'extérieur, formant une ouverture réalisée par exemple à travers la paroi supérieure du boîtier et une deuxième extrémité qui communique avec l'espace formé par la chambre 13. Par ce deuxième canal 15, on peut également injecter des fluides dans ladite chambre ou évacuer des fluides en dehors de ladite chambre. Sa première extrémité est par exemple agencée verticalement et sa deuxième extrémité horizontalement. La chambre 13 est placée entre le premier canal 14 et le deuxième canal 15. De manière identique, la première extrémité de ce deuxième canal est par exemple évasée pour y appliquer le cône d'une pipette ou sera adaptée au type de dispositif employé pour injecter le fluide dans le dispositif. A titre d'exemple, il peut s'agir d'une ouverture présentant un embout de type "luer" pour y connecter une seringue ou adaptée pour y connecter un circuit fluidique tel que celui qui sera décrit ci-après.

Vers le haut, la chambre 13 peut être fermée par une membrane 18 avantageusement souple et étirable, préférentiellement transparente. La paroi supérieure 12 du boîtier du dispositif comporte ainsi une ouverture qui est recouverte de manière hermétique par ladite membrane 18. Ladite membrane est ainsi ancrée dans le boîtier par toute solution de fixation adaptée, par exemple par collage. Cette membrane 18 sera par exemple composée d'un film, par exemple un film autocollant de type PET, d'épaisseur, de dimensions et de constitution adaptées pour se déformer de manière élastique, par rapport à ses points d'ancrage, notamment jusqu'au fond de la chambre 13.

Par le terme "transparent", on entend que le matériau employé est au moins partiellement transparent à la lumière visible, à la fluorescence ou à la luminescence, de manière à laisser passer au moins 80% de cette lumière. Il faut ainsi comprendre qu'il sera suffisamment transparent pour voir l'intérieur de la chambre 13, au moins le deuxième espace situé au-dessus du filtre 16 évoqué ci-dessous.

Le dispositif comporte un filtre 16 agencé dans ladite chambre 13 et séparant ladite chambre 13 en deux espaces. Les deux espaces sont par exemple superposés et désignés ainsi espace inférieur 130 situé sous le filtre et espace supérieur 131 situé au-dessus du filtre et sous la membrane 18. Ce filtre 16 est préférentiellement réalisé en tout ou partie sous la forme d'un film souple et fin, maintenu dans l'espace formé par la chambre de manière à ne permettre le passage d'un espace à l'autre que par les pores du filtre 16. Le film présente avantageusement une déformabilité élastique lui permettant de s'étirer lors de l'exercice d'une force d'appui dans une direction sensiblement verticale, cette déformabilité élastique ayant un niveau suffisant pour atteindre la surface inférieure de la chambre 13. Le filtre 16 présente un diamètre moyen de pores compris entre 10 nm et 50 µm, par exemple compris entre 0.2 µm et 1 µm pour la séparation de microorganismes. Le diamètre des pores est bien entendu adapté pour assurer une séparation entre différentes espèces biologiques présentes dans l'échantillon. Le filtre 16 sera par exemple composé d'un film d'épaisseur, de dimensions et de constitution adaptée pour se déformer jusqu'au fond de la chambre 13 par rapport à ses points d'ancrage. Selon un mode de réalisation particulier, le filtre pourra être aussi réalisé dans un matériau transparent, par exemple avec les mêmes caractéristiques de transparence que la membrane. Pour des bactéries, le filtre peut présenter un diamètre de pores allant de 0.2 à 2µm pour retenir les bactéries.

Si une lyse était nécessaire, par exemple pour libérer des virus intra-cellulaires, le dispositif peut avantageusement comporter une surface d'appui rugueuse 17 agencée sur le fond de la chambre 13. Cette surface d'appui rugueuse 17 s'étend sur une partie majoritaire du fond de la chambre. Elle comporte un paramètre de rugosité de surface moyen compris entre 0.1µm et 10 µm, préférentiellement compris entre 0.2 µm et 3 µm. Cette surface d'appui rugueuse 17 est destinée à permettre une lyse mécanique des espèces biologiques présentes dans un échantillon biologique placé dans le dispositif. Préférentiellement, la lyse mécanique est réalisée en broyant lesdites espèces biologiques, par abrasion sur ladite surface d'appui rugueuse. L'opération de broyage est mise en œuvre par un mouvement de friction des espèces biologiques contre la surface d'appui rugueuse, en employant un organe de broyage adapté. Cet organe sera par exemple une spatule ou une tige, par exemple en matériau plastique ou métallique. Cet organe est appliqué depuis l'extérieur de la chambre 13 et son extrémité est appliquée contre la surface externe de la membrane 18 de manière à étirer la membrane 18 et le filtre vers le fond de la chambre et ainsi frictionner les espèces biologiques présentes dans un échantillon contre la surface d'appui rugueuse 17.

Préférentiellement, le boîtier peut avantageusement intégrer des moyens de chauffage de l'espace interne de la chambre, composés par exemple d'au moins une résistance chauffante. La résistance est par exemple fixée sous la paroi inférieure du boîtier. Une source d'alimentation sera par exemple prévue pour alimenter la résistance. La source d'alimentation comportera par exemple une ou plusieurs piles électriques, fournissant suffisamment d'énergie pour chauffer la chambre à une température comprise dans la fourchette définie ci-dessus, c'est-à-dire de 20°C à 100°C. Bien entendu, d'autres moyens de chauffage pourraient être employés, comprenant par exemple une encre conductrice déposée par imprimerie ou sérigraphie sous la paroi inférieure du boîtier.

Ainsi, pour résumer, le dispositif peut avantageusement comporter la structure "multicouches" suivante :
- Optionnellement, une surface inférieure d'appui rugueuse 17,
- Un espace inférieur 130 de la chambre 13, situé au-dessus de la surface d'appui rugueuse 17,
- Un filtre 16, avantageusement souple et étirable situé au-dessus de l'espace inférieur 130,
- Un espace supérieur 131 de la chambre 13 situé au-dessus du filtre 16,
- Une membrane 18, avantageusement souple et étirable située au-dessus de l'espace supérieur 131, fermant hermétiquement la chambre et accessible depuis l'extérieur du dispositif.

De manière non limitative, le dispositif peut présenter les caractéristiques dimensionnelles suivantes :
- Un premier canal 14 composé d'un canal d'entrée de 1mm de diamètre x 3mm de hauteur, puis d'un canal de section rectangulaire de 1mmx150µm de 3mm de long ;
- Une chambre 13 composé d'un espace inférieur 130 pour concentration/lyse qui présente un diamètre de 8mmx150µm de hauteur et d'un espace supérieur pour élution d'un diamètre 8mmx300µm de hauteur ;
- Filtre 16 de porosité adaptée à la cible à retenir (virus, levure, moisissure...), par exemple avec une porosité de 0.2 à 2 µm pour retenir les bactéries ou de 10 à 100 nm pour retenir les virus ;
- Un deuxième canal 15 composé d'un canal de section rectangulaire de 1mm x 150µm et de 3mm de long, puis d'un canal de 1mm de diamètre x3mm de hauteur ;

Partant de ce dispositif, en référence à la figure 2, le procédé de l'invention présente les étapes suivantes :
E1 : L'échantillon ECH (par exemple sous forme liquide) est injecté dans le dispositif 1 par le premier canal 14, les espèces biologiques E cibles sont maintenues dans l'espace inférieur 130 de la chambre 13 et concentrées dans cet espace.
E2 : Les espèces biologiques E cibles piégées dans l'espace inférieur 130 de la chambre 13 sont lavées avec un tampon L1 adéquat, injecté en flux par le premier canal 14 et évacué par le deuxième canal.
E3 : Les espèces biologiques E cibles sont mises en condition pour la capture sélective. Un tampon T1 adapté est injecté en flux par le premier canal 14 et évacué par le deuxième canal 15 du dispositif.
Les étapes E2 et E3 peuvent être mutualisées et réunies en une seule étape.
E4 : Un réactif R1 hybride adapté à la capture sélective est injecté par le premier canal 14 du dispositif. Ce réactif peut comporter des molécules de capture de type anticorps, aptamère ou MIP (pour "Molecular Imprinted Polymer") ou autres composés connus. Les espèces biologiques E cibles concentrées dans l'espace inférieur 130 de la chambre 13 sont ainsi reconnues par les molécules de capture. Le réactif R1 peut être injecté en flux et reflux, par le premier canal 14 et le deuxième canal 15 en vue d'améliorer la probabilité de rencontre entre les espèces biologiques E cibles et les molécules de capture.
E5 : Il s'agit d'une étape de lavage des espèces capturées dans l'espace inférieur de la chambre, par injection d'un tampon de lavage L2. Cette étape E5 permet de nettoyer les adsorptions non spécifiques de molécules de capture. De même que dans l'étape précédente, Il est possible d'injecter la solution de lavage L2 en aller-retour à travers la chambre 13 du dispositif.
E6 : Il s'agit d'une étape de rinçage avec un tampon T2, adapté pour une mise en condition à la réaction d'amplification biomoléculaire. Le tampon est injecté par le premier canal 14 du dispositif et évacué par le deuxième canal 15.
Les étapes E5 et E6 peuvent être mutualisées et réunies en une seule étape.
E7 : Il s'agit d'une étape d'injection d'un réactif R2 adapté pour la mise en œuvre de la réaction d'amplification.
E8 : La réaction d'amplification se produit. Une partie des amplicons peut migrer à travers le filtre 16 du dispositif. La réaction d'amplification peut nécessiter un chauffage et/ou cyclage du dispositif. La détection peut être réalisée par lecture de la réaction en fluorescence, colorimétrie, pH métrie, imagerie sans lentille. Selon la méthode employée, des courbes d'amplification peuvent être tracées en vue d'obtenir des réponses qualitatives (présence/absence) et quantitative des espèces cibles. Les moyens de détection du système sont adaptés au type de détection mis en œuvre (Matériel optique pour détection de fluorescence, colorimétrie, pH mètre, matériel de détection par imagerie sans lentille...).

Il faut noter que le dispositif doit être adapté au type de détection réalisé. Il peut comporter une ou plusieurs parois transparentes, ainsi que le filtre 16 et la membrane 18. En cas de détection par imagerie sans lentille, la chambre est visible par le dessus et par le dessous, comme illustré par les flèches représentées à l'étape E8 de la figure 2.

Le principe de la détection biomoléculaire peut reposer sur le couplage d'un réactif de reconnaissance de la cible injectée à l'étape E3 avec une séquence oligonucléotidique de préférence de type haltère, ou toute autre séquence oligonucléotidique permettant l'initiation d'une réaction LAMP ("Loop-mediated isothermal AMPlification") ou d'une réaction d'amplification biomoléculaire. Le réactif obtenu correspond ainsi au réactif R1 de type hybride évoqué ci-dessus.

La molécule de reconnaissance de type anticorps (ou toute autre molécule de capture), spécifique de l'espèce biologique E cible à détecter, et mise en œuvre dans la réaction de capture sélective, est liée de manière covalente à une séquence d'oligonucléotides de forme haltère dont le design est tel qu'elle peut être amplifiée de manière isotherme avec au moins deux amorces. Cet haltère est synthétisé préalablement par voie chimique et est conçue de manière à être la plus courte possible (au maximum 200 bases nucléiques) afin d'optimiser sa fabrication et d'accélérer ensuite la réaction d'amplification. Elle peut être identique et générique quelle que soit la cible, et les amorces pour l'amplifier sont spécifiques de cet haltère. Le temps de conception de la réaction biomoléculaire est donc largement raccourci par rapport à une réaction d'amplification classique.

Dans le cas de l'utilisation d'un aptamère comme réactif de reconnaissance, celui-ci peut être directement intégré à la séquence d'amplification en haltère. Dans ce cas, on s'affranchit totalement de l'utilisation d'anticorps, peu stables dans le temps, dont la production est longue et coûteuse et l'utilisation délicate du fait de leur sensibilité importante à la température. On s'affranchit également du couplage de l'haltère avec l'anticorps, dont les étapes de rendement peuvent s'avérer mauvaises. La bonne affinité de l'aptamère envers la cible permet d'assurer la spécificité de la réaction.

Contrairement aux méthodes d'amplification biomoléculaires classiques, celle-ci n'amplifie pas directement l'ADN de la cible, mais une séquence oligonucléotidique contenue dans le réactif R1 hybride permettant la reconnaissance sélective de la cible. . Ainsi, aucune lyse de l'échantillon n'est nécessaire.

L'utilisation d'au moins deux amorces permet d'accélérer nettement la réaction d'amplification. Elle se déroule en moins de 30 minutes à une température constante de 65°C.

Ce principe de réaction est notamment décrit dans la demande de brevet déposée sous le n°FR2002366, déposée le 10/03/2020 (No. publ. FR3108124) et intitulée *"Procédé pour détecter et éventuellement quantifier un analyte avec un oligonucléotide à double structure tige-boucle et ledit oligonucléotide".*

La figure 3 illustre la mise en œuvre du procédé. Sur cette figure 3, on a ainsi :
Etape A : L'échantillon ECH complet qui comporte les espèces biologiques E cibles ainsi que d'autres composants non désirés.
Etape B : Après concentration dans le dispositif à l'étape E2, l'échantillon ne comporte plus que les espèces biologiques E cibles concentrées et partiellement purifiées dans l'espace inférieur 130 de la chambre 13.
Etape C : Il s'agit d'ajouter le réactif R1 hybride de capture sélective, comme prévu à l'étape E4. Les molécules de capture viennent se greffer aux espèces biologiques E cibles.
Etape D : Il s'agit d'ajouter le réactif R2, formé d'amorces AM nécessaires à la réaction d'amplification biomoléculaire, comme prévu à l'étape E7.

Il faut noter qu'une étape de lyse des espèces biologiques présentes dans l'échantillon ECH pourrait également être menée. Cette étape de lyse optionnelle pourrait être mise en œuvre entre l'étape E2 et E3 décrite ci-dessus, c'est-à-dire après l'étape de concentration. Elle serait utile dans le cas où les espèces biologiques E cibles ne seraient pas directement accessibles dans l'échantillon. Dans le cas où l'étape de lyse serait nécessaire, le filtre 16 doit être adapté.

Le procédé de détection de l'invention présente les avantages définis ci-dessous :
- La préparation d'échantillon réalisée de manière commune pour la capture sélective et l'amplification biomoléculaire est très simple, adaptable séquentiellement à chaque étape.
- La préparation de l'échantillon dans le dispositif est très rapide (<10minutes).
- Le procédé ne divise pas l'échantillon : pas de fractionnement pour mettre l'échantillon dans son réactif adéquat, donc pas de dilution.
- L'intégralité des espèces cibles présentes dans l'échantillon est engagée dans la réaction de détection (pas de dilution, d'utilisation d'une partie de l'éluât...).
- Chaque étape est réalisée directement dans son réactif (pas de dilution ni fractionnement de l'échantillon pour le préparer et le détecter).
- Cette méthode de préparation d'échantillons permet de concentrer, de purifier et ne comporte avantageusement pas d'étape de lyse.
- La sensibilité est comparable ou supérieure aux méthodes de référence en immunologie ou en PCR.
- La spécificité est différente selon la cible, la souche, le type de récepteur...
- Elle est mise en œuvre à partir d'un unique dispositif pour l'enchaînement capture sélective et détection biomoléculaire.

## Revendications

1. Procédé de détection d'espèces biologiques cibles dans un échantillon biologique, ledit procédé étant mis en œuvre dans un système de détection qui comporte un dispositif de préparation et des moyens de détection, ledit dispositif de préparation comportant :
- Un boîtier comportant au moins une ouverture,
- Un premier canal (14) ménagé dans ledit boîtier,
- Un deuxième canal (15) ménagé dans ledit boîtier,
- Une chambre (13) dans laquelle débouche le premier canal et le deuxième canal,
- Un filtre (16) séparant ladite chambre en deux espaces distincts de manière à définir un premier espace (130) dans lequel débouche ledit premier canal d'injection et un deuxième espace (131) dans lequel débouche ledit deuxième canal, ledit filtre (16) ayant une porosité adaptée pour retenir ledit matériel biologique à analyser,
Ledit procédé étant **caractérisé en ce qu'**il comporte des étapes de :
- Injection de l'échantillon par le premier canal pour effectuer une concentration des espèces biologiques cibles dans le premier espace de la chambre, mise en œuvre par filtration,
- Injection par le premier canal d'une première solution de mise en condition de réaction par reconnaissance sélective,
- Injection par le premier canal d'une solution d'un premier réactif (R1) hybride comportant des molécules de reconnaissance sélective des espèces biologiques cibles, ces molécules de reconnaissance sélective étant liées à une séquence oligonucléotidique qui est adaptée à une initiation d'une réaction d'amplification biomoléculaire,
- Injection par le premier canal (14) d'une deuxième solution de mise en condition de réaction par amplification biomoléculaire,
- Injection par le premier canal (14) d'une solution d'un réactif (R2) adapté à une amplification biomoléculaire,
- Détection des espèces cibles directement dans le dispositif (1) par amplification biomoléculaire de la séquence oligonucléotidique des molécules de reconnaissance sélective.

2. Procédé selon la revendication 1, **caractérisé en ce qu'**il comporte une étape de lavage des espèces biologiques cibles concentrées dans l'espace inférieur du dispositif, après l'étape d'injection de l'échantillon.

3. Procédé selon la revendication 1 ou 2, **caractérisé en ce qu'**il comporte une étape de lavage des espèces biologiques cibles mise en œuvre après la réaction de reconnaissance sélective.

4. Procédé selon l'une des revendications 1 à 3, **caractérisé en ce que** l'étape de détection par amplification est mise en œuvre par fluorescence, colorimétrie, pH métrie, turbidimétrie, imagerie sans lentille.

5. Procédé selon l'une des revendications 1 à 4, **caractérisé en ce que** le premier réactif (R1) comporte des molécules de capture de type anticorps ou aptamère ou de type MIP.

6. Procédé selon l'une des revendications 1 à 5, **caractérisé en ce que** le deuxième réactif comporte des amorces (AM) de réaction par amplification.

## Patentansprüche

1. Verfahren zur Detektion von biologischen Zielspezies in einer biologischen Probe, wobei das Verfahren in einem Detektionssystem durchgeführt wird, das eine Vorbereitungsvorrichtung und Detektionsmittel umfasst, wobei die Vorbereitungsvorrichtung Folgendes umfasst:
- Ein Gehäuse, das mindestens eine Öffnung umfasst,
- Einen ersten Kanal (14), der in dem Gehäuse ausgebildet ist,
- Einen zweiten Kanal (15), der in dem Gehäuse ausgebildet ist,
- Eine Kammer (13), in die der erste Kanal und der zweite Kanal münden,
- Einen Filter (16), der die Kammer in zwei verschiedene Räume teilt, so dass ein erster Raum (130) definiert wird, in den der erste Einleitungskanal mündet, und ein zweiter Raum (131), in den der zweite Kanal mündet, wobei der Filter (16) eine Porosität aufweist, die geeignet ist, das zu analysierende biologische Material zurückzuhalten,
Wobei das Verfahren **dadurch gekennzeichnet ist, dass** es die folgenden Schritte umfasst:
- Einleiten der Probe durch den ersten Kanal, um ein Konzentrieren der biologischen Zielspezies in dem ersten Raum der Kammer vorzunehmen, das durch Filtration durchgeführt wird;
- Einleiten einer ersten Lösung zur Konditionierung für die Reaktion durch selektive Erkennung durch den ersten Kanal,
- Einleiten einer Lösung eines ersten hybriden Reagenzes (R1), das Moleküle zur selektiven Erkennung der biologischen Zielspezies umfasst, durch den ersten Kanal, wobei diese Moleküle zur selektiven Erkennung an eine Oligonukleotid-Sequenz gebunden sind, die für eine Initiierung einer Reaktion zur biomolekularen Amplifikation geeignet ist,
- Einleiten einer zweiten Lösung zur Konditionierung für die Reaktion durch biomolekulare Amplifikation durch den ersten Kanal (14),
- Einleiten einer Lösung eines Reagenzes (R2), das für eine biomolekulare Amplifikation geeignet ist, durch den ersten Kanal (14),
- Detektion der Zielspezies direkt in der Vorrichtung (1) durch biomolekulare Amplifikation der Oligonukleotid-Sequenz der Moleküle zur selektiven Erkennung.

2. Verfahren nach Anspruch 1, **dadurch gekennzeichnet, dass** es einen Schritt des Waschens der im unteren Raum der Vorrichtung konzentrierten biologischen Zielspezies nach dem Schritt des Einleitens der Probe umfasst.

3. Verfahren nach Anspruch 1 oder 2, **dadurch gekennzeichnet, dass** es einen Schritt des Waschens der biologischen Zielspezies umfasst, der nach der Reaktion zur selektiven Erkennung durchgeführt wird.

4. Verfahren nach einem der Ansprüche 1 bis 3, **dadurch gekennzeichnet, dass** der Schritt der Detektion durch Amplifikation durch Fluoreszenz, Kolorimetrie, pH-Wert-Messung, Trübungsmessung oder linsenlose Bildgebung durchgeführt wird.

5. Verfahren nach einem der Ansprüche 1 bis 4, **dadurch gekennzeichnet, dass** das erste Reagenz (R1) Fängermoleküle vom Typ Antikörper oder Aptamer oder vom Typ MIP umfasst.

6. Verfahren nach einem der Ansprüche 1 bis 5, **dadurch gekennzeichnet, dass** das zweite Reagenz Primer (PM) für die Amplifikationsreaktion umfasst.

## Claims

1. Method of detecting target biological species in a biological sample, said method being performed in a detection system which comprises a preparation device and detection means, said preparation device comprising:
- a housing having at least one opening,
- a first canal (14) formed in said housing,
- a second canal (15) formed in said housing,
- a chamber (13) into which the first canal and the second canal open,
- a filter (16) dividing said chamber into two distinct spaces so as to define a first space (130) into which said first injection canal opens and a second space (131) into which said second canal opens, said filter (16) having a porosity suitable for retaining said biological material that is to be analysed,
said method being **characterized in that** it comprises steps of:
- injecting the sample through the first canal to achieve a concentration of the target biological species in the first space of the chamber, this being achieved by filtration,
- injecting, through the first canal, a first solution in preparation for selective recognition reaction,
- injecting, via the first canal, a solution of a first hybrid reagent (R1) comprising molecules for selective recognition of the target biological species, these selective recognition molecules being linked to an oligonucleotide sequence which is suitable for initiation of a biomolecular amplification reaction,
- injecting, through the first canal (14), a second solution in preparation for biomolecular amplification reaction,
- injecting, through the first canal (14), a solution of a reagent (R2) suitable for biomolecular amplification,
- detecting target species directly in the device (1) by biomolecular amplification of the oligonucleotide sequence of the selective recognition molecules.

2. Method according to Claim 1, **characterized in that** it comprises a step of washing the target biological species concentrated in the lower space of the device, after the step of injecting the sample.

3. Method according to Claim 1 or 2, **characterized in that** it comprises a step of washing the target biological species, this step being performed after the selective recognition reaction.

4. Method according to one of Claims 1 to 3, **characterized in that** the step of detection by amplification is performed by fluorescence, colorimetry, measuring pH, turbidimetry or lensless imaging.

5. Method according to one of Claims 1 to 4, **characterized in that** the first reagent (R1) comprises capture molecules of antibody or aptamer type or of MIP type.

6. Method according to one of Claims 1 to 5, **characterized in that** the second reagent comprises amplification-reaction primers (AM).
